# FASCICULE DE BREVET EUROPEEN

(11) **EP 1 473 017 B1**
(45) Date de publication et mention de la délivrance du brevet: **23.03.2011**
(21) Numéro de dépôt: 04290858.2
(22) Date de dépôt: 01.04.2004
(51) Int. Cl.: A61K 8/891, A61K 8/97, A61K 8/06, A61K 8/86, A61K 8/92, A61Q 1/06, A61Q 1/08, A61Q 1/10, A61Q 1/02, A61Q 19/00, A61Q 17/04, A61Q 19/08, A61Q 19/04

(54) **Emulsion eau-dans-huile solide utilisable en cosmétique**
Feste Öl-in-Wasser Emulsion die für die Kosmetik geeignet ist
Solid oil-in-water emulsion suitable for use in cosmetics

(30) Priorité: 28.04.2003 FR 0305175
(43) Date de publication de la demande: 03.11.2004
(73) Titulaire: L'Oréal, 75008 Paris (FR)
(72) Inventeur: Verloo, Aurore, 91570 Bievres (FR); Fontaine, Jacqueline, 78170 La Celle Saint Cloud (FR)
(74) Mandataire: Kromer, Christophe

(56) Documents cités:
- WO-A-99/49878
- FR-A- 2 776 183
- US-A- 4 411 887
- US-A- 5 968 484
- US-A- 6 132 703
- US-A1- 2002 082 327

## Description

La présente invention a pour objet une émulsion solide eau-dans-huile comprenant une cire et un copolymère particuliers, utilisable dans le domaine cosmétique. L'invention a également pour objet un procédé de maquillage ou de soin de la peau d'être humain comprenant l'application de la composition sur la peau.

La composition de maquillage selon l'invention est en particulier une composition de maquillage de la peau, telle qu'un fond de teint, un fard à paupières, un fard à joue, un produit anticernes, un produit de maquillage du corps, un rouge à lèvres. Plus spécialement, l'invention porte sur une composition de fond de teint.

La composition de soin peut être un produit de soin de la peau tels qu'une base de soin pou la peau, une crème de soin (crème de jour, de nuit, anti-rides), une base de maquillage ; une composition de soin pour les lèvres (baume à lèvres) ; une composition de protection solaire ou autobronzante ; un déodorant.

Les produits de maquillage de la peau comme les fonds de teint sont connus sous formes de galéniques très diverses : poudre libre, poudre compacte, produit solide coulé, stick, crème fluide. Les poudres compactes ont généralement un aspect très mat, tandis que les crèmes fluides ont plutôt un aspect brillant.

Les produits solides coulés peuvent être anhydres ou bien sous forme d'émulsions. Ces dernières contiennent généralement des corps gras tels que des huiles et des cires solides, de l'eau et une phase particulaire généralement composée de charges et de pigments, comme décrit par exemple dans la demande WO 99/47111.
L' émulsion solide ne s'écoule pas sous son propre poids à la température ambiante et est adaptée pour être conditionnée dans un boîtier : pour appliquer le produit, l'utilisatrice peut prendre directement le produit en le délitant à l'aide des doigts ou d'un applicateur tel qu'une éponge.

Par ailleurs, la présence de cire dans l'émulsion solide à tendance à opacifier le produit, ce dernier présentant alors un aspect mat en surface. De plus, lorsque l'utilisatrice prend du produit avec les doigts ou avec une éponge en délitant la surface de la composition solide, elle ressent un certain frein lors du délitage : le produit ne présente pas un glissant suffisant pour faciliter une prise rapide du produit.

Le but de la présente invention est donc de disposer d'une composition solide de maquillage ou de soin de la peau présentant un bon glissant lors de la prise de produit avec les doigts ou avec une éponge, facilitant une prise rapide du produit lors de son délitage.

Un autre but de l'invention est de disposer d'une composition solide présentant un aspect brillant lors de son délitage.

Les inventeurs ont découvert qu'en utilisant un copolymère d'oxyde d'alkylène particulier dans une émulsion solide comprenant des cires, il est possible d'obtenir un produit solide présentant un bon glissant lors de son délitage et permettant à l'utilisatrice une bonne prise du produit aux doigts ou à l'éponge. De plus, le produit avant son utilisation présente un aspect mat en surface, aspect qui devient brillant lors de son délitage : ainsi le produit solide présente un aspect miroir pendant le délitage ; cet effet visuel miroir rend très attrayant le produit aux utilisatrices.

De façon plus précise, l'invention a pour objet une émulsion solide eau-dans-huile comprenant une phase aqueuse émulsionnée avec un tensioactif émulsionnant dans une phase grasse comprenant une huile et une cire, caractérisée par le fait que la phase grasse comprend un copolymère séquence d'oxyde d'éthylène et/ou d'oxyde de propylène et d'oxyde d'alkylène ayant de 6 à 40 atomes de carbones, le copolymère ayant un poids moléculaire moyen en poids allant de 5000 à 8000 et que l'émulsion a une dureté telle que la force de pénétration supérieure ou égale à 40 g.

L'invention a également pour objet un procédé cosmétique de maquillage ou de soin non thérapeutique de la peau comprenant l'application sur la peau d'une composition telle que définie précédemment.

On entend par composition solide une composition qui ne s'écoule pas sous son propre poids à température ambiante (25 °C) au bout d'une heure.

L'émulsion selon l'invention comprend un copolymère séquence d'oxyde d'éthylène et/ou d'oxyde de propylène et d'oxyde d'alkylène ayant de 6 à 40 atomes de carbones, le copolymère ayant un poids moléculaire moyen en poids allant de 5000 à 8000.

Avantageusement, l'oxyde d'alkylène du copolymère peut comprendre de 6 à 30 atomes de carbone, de préférence de 8 à 20 atomes de carbone, préférentiellement de 10 à 18 atomes de carbone, et plus préférentiellement de 10 à 14 atomes de carbone.

Le poids moléculaire moyen en poids du copolymère va de 5000 à 8000, de préférence de 5500 à 7000, préférentiellement de 5500 à 6500, et plus préférentiellement de 5800 à 6200.

Le copolymère comprend avantageusement de 35 à 55 motifs d'oxyde d'éthylène et/ou d'oxyde de propylène et de 15 à 30 motifs d'oxyde d'alkylène ayant de 6 à 40 atomes de carbones.

En particulier, le copolymère est tel que le rapport entre le nombre de motifs d'oxyde d'éthylène et/ou d'oxyde de propylène et le nombre de motifs d'oxyde d'alkylène ayant de 6 à 40 atomes de carbones peut aller de 1,5 à 2,5, de préférence aller de 1,8 à 2,3 et préférentiellement aller de 1,9 à 2,1.

De tels copolymères sont notamment décrits dans le document FR-A-2425848 et vendus sous la dénomination "ELFACOS® ST 9" par la société AKZO NOBEL.

Ledit copolymère peut être présent dans la composition selon l'invention en une teneur allant de 1 % à 25 % en poids, par rapport au poids total de la composition, de préférence allant de 1 % à 18 % en poids, et préférentiellement allant de 1 % à 10 % en poids.

La phase grasse de l'émulsion selon l'invention comprend au moins une huile qui peut être choisie parmi les huiles d'origine minérale, animale, végétale ou synthétique, carbonées, hydrocarbonées, fluorées et/ou siliconées, seules ou en mélange.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Par huile siliconée, on entend une huile contenant au moins un atome de silicium, et notamment contenant des groupes Si-O.

Par huile fluorée, on entend une huile contenant au moins un atome de fluor.

Avantageusement, l'émulsion peut comprendre une huile volatile.
Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg) .

En outre, l'huile volatile a généralement un point d'ébullition, mesuré à pression atmosphérique, allant de 150 °C à 260 °C, et de préférence allant de 170 °C à 250 °C.

Comme huile organique volatile utilisable dans l'invention, on peut citer :
- les huiles de silicones linéaires ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane ;
- les huiles volatiles hydrocarbonées ayant de 8 à 16 atomes de carbone et leurs mélanges et notamment les alcanes ramifiés en C₈-C₁₆ comme les isoalcanes (appelées aussi isoparaffines) en C₈-C₁₆, l'isododécane, l'isodécane, l'isohexadécane et par exemple les huiles vendues sous les noms commerciaux d'Isopars' ou de Permethyls', les esters ramifiés en C₈-C₁₆ comme le néo pentanoate d'isohexyle et leurs mélanges ; on utilise de préférence l'isododécane ;
- les huiles fluorées volatiles telles que le nonafluoroéthoxybutane, le nonafluorométhoxybutane, le décafluoropentane, le tétradécafluorohexane, le dodécafluoropentane, et leurs mélanges.
- et leurs mélanges.

De préférence, l'émulsion selon l'invention comprend au moins une huile volatile siliconée.

Avantageusement, l'huile volatile peut être présente en une teneur allant de 0,1 % à 35 % en poids, par rapport au poids total de la composition, de préférence allant de 1 % à 30 % en poids, et préférentiellement allant de 5 % à 25 % en poids.

La composition peut comprendre, en outre, une huile non volatile.

Comme huiles non volatiles utilisables dans l'invention, on peut citer les huiles hydrocarbonées d'origine minérale ou synthétique telles les hydrocarbures linéaires ou ramifiés comme l'huile de paraffine ou ses dérivés, l'huile de vaseline, les polydécènes, le polyisobutène hydrogéné tel que le Parléam commercialisé par la société Nippon Oil Fats, le squalane d'origine synthétique ou végétale ; les huiles d'origine animale comme l'huile de vison, de tortue, le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, notamment les triglycérides d'acide gras notamment de 4 à 22 atomes de carbone, comme les triglycérides des acides heptanoïque, octanoïque, et des acides caprique/caprylique ou bien encore les triglycérides hydroxylés, telles que l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive, de germes de céréales (maïs, blé, orge, seigle), de beurre de karité ;
des esters d'acide gras , en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le polyglycéryl 2 diisostéarate, le diheptanoate de néopentylglycol ;
les esters de synthèse de formule R₁COOR₂ dans laquelle R₁ représente le reste d'un acide gras supérieur linéaire ou ramifié comportant de 7 à 40 atomes de carbone et R₂ représente une chaîne hydrocarbonée ramifiée contenant de 3 à 40 atomes de carbone, comme par exemple l'huile de Purcellin (octanoate de cétostéaryle), l'isononanoate d'isononyle, le benzoate d'alcool en C₁₂ à C₁₅, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyldodécyle, le succinate de 2-diéthylhexyle, le malate de diisostéaryle, le néopentanoate d'isodécyle ; les esters hydroxylés comme le lactate d'isostéaryle, l'octylhydroxystéarate, l'hydroxystéarate d'octyldodécyle, le diisostéarylmalate, le citrate de triisocétyle, le triisostéarate de glycérine ou de diglycérine; le diisononanoate de diéthylèneglycol ; les esters du pentaérythritol ; les esters d'acides aromatiques et d'alcools comprenant 4 à 22 atomes de carbone, notamment le trimellitate de tridécyle ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alcoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols ; les silicones fluorées liquides ;
et leurs mélanges.

Avantageusement, l'huile non volatile peut être présente en une teneur allant de 0,1 % à 35 % en poids, par rapport au poids total de la composition, de préférence allant de 1 % à 30 % en poids, et préférentiellement allant de 5 % à 25 % en poids.

L'émulsion selon l'invention a une dureté telle que la force de pénétration supérieure ou égale à 40 grammes (g), notamment allant de 40 g à 150 g.

La force de pénétration de l'émulsion est mesurée selon le protocole suivant :

En fin de préparation de l'émulsion, cette dernière est coulée dans une coupelle et est maintenue 24 heures à 20 °C. On mesure alors sur cette émulsion solide la force de pénétration à l'aide d'un appareil analyseur de texture de marque STEVENS, au mobile de mesure TA24, diamètre de 4 mm, à une vitesse de pénétration de 0,5 mm/s et à la profondeur de pénétration présélectionnée de 2 mm. La force de pénétration exprimée en grammes se lit sur l'appareil.

De préférence, l'émulsion solide a une dureté telle que la force de pénétration va de 40 g à 150 g, de préférence va de 50 g à 120 g, préférentiellement va de 50 g à 90 g, et plus préférentiellement va de 60 g à 80 g.

Avantageusement, la cire présente dans l'émulsion selon l'invention peut être choisie parmi les cires ayant une dureté allant de 5MPa à 9 MPa, et de préférence allant de 6 MPa à 9 MPa, et préférentiellement allant de 7 MPa à 9 MPa.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

La dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Les cires, au sens de la demande, peuvent être hydrocarbonées, siliconées et/ou fluorées, comportant éventuellement des fonctions ester ou hydroxyle.

La cire ayant la dureté telle que définie précédemment peut être choisie parmi la cire de Carnauba, les cires microcristallines, les ozokérites, l'huile de jojoba hydrogénée, les cires de polyéthylène telles que celle vendue sous les dénominations "PERFORMALENE 400 POLYETHYLENE" par la société New Phase Technologies, les cires de silicone comme les poly alkyle C24-C28 méthyl dimethyl siloxane telle que celle vendue sous la dénomination "ABIL WAX 9810" par la société GOLDSCHMIDT, le beurre de palme, le stéarate d'alkyle en C20-C40 vendu sous la dénomination "KESTER WAX K82H" par la société Kester Keunen, le benzoate de stéaryle, la cire de Shellac, et leurs mélanges. On utilise de préférence une cire choisie parmi la cire de Carnauba, les ozokérites, l'huile de jojoba hydrogénée, les cires de polyéthylène.

La cire peut être présente dans la composition selon l'invention en une teneur allant de 1 % à 10 % en poids, par rapport au poids total de la composition, de préférence allant de 2 % à 7 % en poids, et préférentiellement allant de 2,5 % à 5 % en poids.

La phase aqueuse de l'émulsion solide selon l'invention comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants miscibles à l'eau (à température ambiante - 25 °C) comme par exemple les alcools primaires en C₂-C₄ tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

La phase aqueuse peut comprendre en outre des agents de stabilisation , par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse est présente dans l'émulsion selon l'invention en une teneur allant 30 % à 50 %, en poids, de préférence allant de 35 % à 45 % en poids, par rapport au poids total de l'émulsion.

L'émulsion selon l'invention comprend un tensioactif émulsionnant permettant l'obtention d'émulsion eau-dans-huile, notamment un tensioactif ayant un HLB (balance hydrophile/lipophile) inférieur à 7 ; un tel tensioactif émulsionnant peut être choisi parmi les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone ; les polymères du type ester d'acide gras de glycol polyoxyalkyléné.

En particulier, le tensioactif émulsionnant peut être un alkyl C₈-C₂₂ diméthicone copolyol, c'est-à-dire un poly méthyl alkyl(C₈-C₂₂) diméthyl méthyl siloxane oxypropyléné et/ou oxyéthyléné.

L'alkyl C₈-C₂₂ diméthicone copolyol est avantageusement un composé de formule (1) suivante : 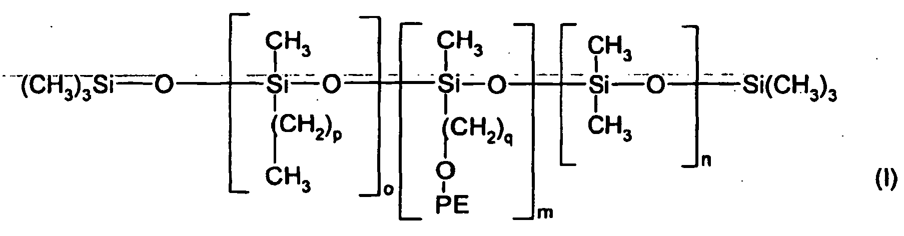 dans laquelle :
- PE représente (-C₂H₄O)ₓ (C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanémént 0
- m allant de 1 à 40
- n allant de 10 à 200
- o allant de 1 à 100
- p allant de 7 et 21
- q allant de 0 à 4
et de préférence :
R=H
m=1 à 10
n= 10 à 100
o = 1 à 30
p = 15
q=3

Comme alkyl C₈-C₂₂ diméthicone copolyol, on peut citer le cétyl diméthicone copolyol comme le produit commercialisé sous la dénomination Abil EM-90 par la société Goldschmidt.

Comme autre tensioactif utilisable dans l'invention pour l'obtention d'une émulsion E/H on peut citer les polymères du type ester d'acide gras de glycol polyoxyalkyléné ayant des propriétés émulsionnantes eau-dans-huile.

L'ester d'acide gras dudit polymère est de préférence polyhydroxylé. En particulier, ce polymère est un polymère séquence, de préférence de structure ABA, comportant des séquences poly(ester hydroxylé) et des séquences polyéthylèneglycols.

L'ester d'acide gras dudit polymère émulsionnant tel que défini ci-dessus présente en général une chaîne comportant de 12 à 20 atomes de carbone, de préférence de 14 à 18 atomes de carbone. Les esters peuvent notamment être choisis parmi les oléates, les palmitates ou les stéarates.

Les séquences polyéthylèneglycols dudit polymère émulsionnant tel que défini ci-dessus comportent de préférence de 4 à 50 moles d'oxyde d'éthylène, et de préférence encore de 20 à 40 moles d'oxyde d'éthylène.

Un tensioactif polymère convenant particulièrement à la réalisation des compositions de l'invention est le di-polyhydroxystéarate de polyéthylène glycol à 30 OE vendu sous la dénomination commerciale « Arlacel P 135 » par la société ICI.

Le tensioactif émulsionnant peut être présent dans la composition en une teneur allant de 1 % à 10 % en poids, par rapport au poids total de la composition, et de préférence allant de 2 % à 5 % en poids.

L'émulsion selon l'invention peut comprendre au moins une charge.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®) , de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsuies de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes dans la composition en une teneur allant de 0,1 % à 25 % en poids, par rapport au poids total de la composition, de préférence allant de 1 % à 15 % en poids.

L'émulsion selon l'invention peut comprendre au moins une matière colorante pouvant être choisie parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la composition.
Par « nacres », il faut comprendre des particules irisées, notamment produites par certains mollusques dans leur coquille ou bien synthétisées, insolubles dans le milieu de la composition.
Par « colorants », il faut comprendre des composés généralement organiques solubles dans les corps gras comme les huiles ou dans une phase hydroalcoolique.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.
On utilise de préférence des pigments d'oxydes de fer ou de dioxyde de titane.

Avantageusement, les pigments sont traités avec un agent hydrophobe pour les rendre compatible avec la phase organique de la composition. L'agent de traitement hydrophobe peut ëtre choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.
Les colorants hydrosolubles sont par exemple le jus de betterave, le bleu de méthylène, le caramel.

Les matières colorantes peuvent être présentes en une teneur allant de 0,5 % à 30 % en poids, par rapport au poids total de la composition, de préférence allant de 3 % à 20 % en poids, et préférentiellement allant de 5 à 15 % en poids.

La composition peut contenir d'autres ingrédients cosmétiques usuels pouvant être choisis notamment parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut être préparé selon le mode opératoire suivant :
On prépare d'une part le mélange des constituants de la phase huileuse en mélangeant et en chauffant à une température comprise entre 70 °C et 120 °C les cires, le copolymère séquence d'oxyde d'éthylène et/ou d'oxyde de propylène et d'oxyde d'alkylène ayant de 6 à 40 atomes de carbones, les huiles non volatiles, puis en ajoutant sous agitation, à une température comprise entre 60 °C et 80 °C, les huiles volatiles, les charges et les pigments.

On prépare d'autre part le mélange des constituants de la phase aqueuse comprenant l'eau, les tensioactifs, et les solvants miscibles à l'eau en chauffant à une température comprise entre 60 °C et 80 °C.

Puis on ajoute la phase aqueuse dans la phase huileuse, à une température comprise entre 60°C et 80°C, et on agite à l'aide d'une turbine jusqu'à l'obtention de l'émulsion eau-dans-huile. L'émulsion est ensuite coulée dans un contenant, par exemple une coupelle, puis refroidie à température ambiante jusqu'à l'obtention de l'émulsion solide.

L'invention est illustrée plus en détails par les exemples décrits ci-après.

### Exemple 1 :

On a préparé un fond de teint solide ayant la composition suivante :

### Phase huileuse :

| | |
|---|---|
| Copolymère d'oxyde d'éthylène (45 OE) et d'époxydodécane (22 moles) vendu sous la dénomination "ELFACOS® ST 9" par la société AKZO NOBEL | 4 g |
| Huile de jojoba hydrogénée | 2,7 g |
| Ozokérite | 1 g |
| Néopentanoate d'isodécyle | 9,5 g |
| Cyclopentasiloxane | 22,5 g |
| Oxydes de fer enrobés par sel disodique de stéaroyl glutamate et d'aluminium | 2,4 g |
| Dioxyde de titane enrobés par sel disodique de stéaroyl glutamate et d'aluminium | 7,6 g |
| Silice | 9,5 g |
| Polyméthacrylate de méthyle | 3 g |
| Nanopigments de dioxyde de titane | 3 g |

### Phase aqueuse :

| | |
|---|---|
| Eau | 20 g |
| Butylène glycol | 3 g |
| Glycérine | 5 g |
| Sulfate de magnésium | 1 g |
| Diisostéarate de polyglycéryle-3 | 0,3 g |
| Cétyl diméthicone copolyol (Abil® EM 90 de la société GOLDSCHMIDT) Mélange de polydiméthylsiloxane oxyéthyléné oxypropyléné (18 OE/18 OP), de cyclopentasiloxane et d'eau (10188/2) | 2 g |
| (DC 2-5225 C de DOW CORNING) | 2 g |

La composition a été préparée selon le mode opératoire suivant :
On chauffe à 90 °C le mélange comprenant les cires, le copolymère ELFACOS ST 9 et les huiles non volatiles jusqu'à obtention d'un mélange homogène et limpide ; puis on ajoute sous agitation à 70 °C les huiles volatiles, les pigments et les charges. On prépare ensuite le mélange des ingrédients de la phase aqueuse en chauffant à 70 °C, puis la phase aqueuse est introduite à 70 °C dans la phase huileuse, sous agitation jusqu'à obtention de l'émulsion eau-dans-huile.

L'émulsion est coulée à 70 °C dans une coupelle préalablement chauffée puis on laisse refroidir à la température ambiante jusqu'à obtention de l'émulsion solide.

On obtient un fond de teint solide ayant une dureté de 60 g mesurée à 24 heures à 20 )C selon le protocole précédemment décrit, qui présente une surface glacée (effet miroir) lorsque l'on délite la composition avec les doigts ; le fond de teint fond agréablement sur la peau lors de l'application.

## Revendications

1. Emulsion solide eau-dans-huile comprenant une phase aqueuse émulsionnée avec un tensioactif émulsionnant alkyl C₈-C₂₂ diméthicone copolyol de formule (l) suivante : 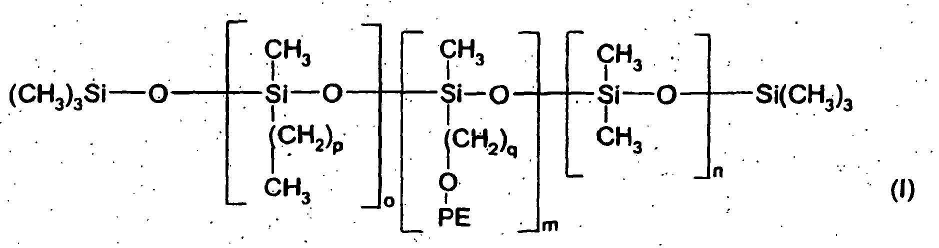 dans laquelle:
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanément 0
- m allant de 1 à 40
- n allant de 10 à 200
- o allant de 1 à 100
- p allant de 7 et 21
- q allant de 0 à 4,
ledit tensioactif étant présent dans une phase grasse comprenant une huile et une cire, **caractérisée par le fait que** la phase grasse comprend un copolymère séquencé d'oxyde d'éthylène et/ou d'oxyde de propylène et d'oxyde d'alkylène ayant de 6 à 40 atomes de carbones, le copolymère ayant un poids moléculaire moyen en poids allant de 5000 à 8000 et que l'émulsion a une dureté telle que la forcé de pénétration est supérieure ou égale à 40 g.

2. Emulsion selon la revendications 1, **caractérisée par le fait que** l'oxyde d'alkylène du copolymère séquencé comprend de 6 à 30 atomes de carbone, de préférence de 8 à 20 atomes de carbone, préférentiellement de 10 à 18 atomes de carbone, et plus préférentiellement de 10 à 14 atomes de carbone.

3. Emulsion selon la revendication 1 ou 2, **caractérisée par le fait que** l'oxyde d'alkyléne du, copolymère comprend de 6 à 30 atomes de carbone, de préférence de 8 à 20 atomes de carbone, préférentiellement de 10 à 18 atomes de carbone, et plus préférentiellement de 10 à 14 atomes de carbone.

4. Emulsion selon l'une quelconque des revendications précédentes, **caractérisée par le fait que** le poids moléculaire moyen en nombre du copolymère va de 5000 à 8000, de préférence de 5500 à 7000, préférentiellement de 5500 à 6500 et plus préférentiellement de 5800 à 6200.

5. Emulsion selon l'une quelconque des revendications précédentes, **caractérisée par le fait que** le copolymère comprend de 35 à 55 motifs d'oxyde d'éthylène et/ou d'oxyde de propylène et de 15 à 30 motifs d'oxyde d'alkylène ayant de 6 à 40 atomes de carbonés.

6. Emulsion selon l'une quelconque des revendications précédentes, **caractérisée, par le fait que** le copolymères est tel que le rapport entre le nombre de motifs d'oxyde d'éthylène et/ou d'oxyde de propylène et le nombre de motifs d'oxyde d'alkylène ayant de 6 à 40 atomes de carbones va de 1,5 à 2,5, de préférence va de 1,8 à 2,3 et préférentiellement va de 1,9 à 2,1.

7. Emulsion selon l'une quelconque des revendications précédentes, **caractérisée par le fait que** le copolymère est présent en une teneur allant de 1 % à 25 % en poids, par rapport au poids total de la composition, de préférence allant de 1 % à 18 % en poids, et préférentiellement allant de 1% à 10% en poids.

8. Emulsion selon l'une quelconque des revendications précédentes, **caractérisée par le fait qu'**elle a une dureté telle que la force de pénétration va de 40 g à 150 g, de préférence va, de 50 g à 120 g, préférentiellement va de 50 g à 90 g, et plus préférentiellement va de 60 g à 80 g.

9. Emulsion selon l'une quelconque des revendications précédentes, **caractérisée par le fait que** la phase grasse comprend une huile volatile.

10. Emulsion selon la revendication 9. **caractérisée par le fait que** l'huile volatile est choisie parmi l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane, l'isododécane, l'isodécane, l'isohexadécane, et leurs mélanges.

11. Emulsion selon la revendication 9 ou 10, **caractérisée par le fait que** l'huile volatile est présente en une teneur allant de 0,1 % à 35% en poids, par rapport au poids total de la composition, de préférence allant de 1% à 30 %en poids, et préférentiellement allant de 5 % à 25 % en poids.

12. Emulsion selon l'une quelconque des revendications précédentes, **caractérisée par le fait que** la phase grasse comprend une huile non volatile.

13. Emulsion selon la revendication 12, **caractérisée en ce que** l'huile non volatile est présente en une teneur allant de 0,1 % à 35.% en poids, par-rapport au poids total de la composition, de préférence allant de 1 % à 30 % en poids, et préférentiellement allant de 5 % à 25 % en poids.

14. Emulsion selon l'une quelconque des revendication précédentes, **caractérisée par le fait que** la cire a une dureté allant de 5 MPa à 9 MPa.

15. Emulsion selon l'une quelconque des revendications précédentes, **caractérisée par le fait que** la cire a une dureté allant de 6 MPa à 9 MPa, et de préférence allant de 7 MPa à 9 MPa.

16. Mulsion selon l'une quelconque des revendications précédentes, **caractérisée par le fait que** la cire est choisie parmi la cire de Carnauba, les cires microcristallines, les ozokérites, l'huile de jojoba hydrogénée, les cires de polyéthylène, les cires de poly alkyle C24-C28 méthyl dimethyl siloxane, le beurre de palme, le stéarate d'alkyle en C20-C40, le benzoate de stéaryle, la cire de Shellac, et leurs mélanges.

17. Emulsion selon l'une quelconque des revendications précédentes, **caractérisée par** te fait que la cire est choisie parmi la cire de Carnauba, les ozokérites, l'huile, de jojoba hydrogénée, les cires de polyéthyléne.

18. Emulsion selon l'une quelconque des revendications précédentes, **caractérisée par le fait que** la cire est présente en une teneur allant de 1 % à 10 % en poids, par rapport au poids total de la composition, de préférence allant de 2 % à 7 % en poids, et préférentiellement allant de 2,5 % à 5 % en poids.

19. Emulsion selon l'une quelconque des revendications précédentes, **caractérisé par le fait que** la phase aqueuse est présente en une teneur allant 30 % à 50 %, en poids, de préférence allant de 35 % à 45 % en poids, par rapport au poids total de l'émulsion.

20. Fond de teint selon l'une quelconque des revendications précédentes, **caractérisé par le fait que** la phase aqueuse comprend un solvant miscible à l'eau, notamment choisi parmi les alcools primaires en C₂-C₄, les glycols, les éthers de glycol, et leurs mélanges.

21. Emulsion selon une quelconque des revendications précédentes, **caractérisée par le fait que** le tensioactif émulsionnant est présent en une teneur allant de 1 % à 10 % en poids; par rapport au poids total de la composition, et de préférence allant de 2 % à5% en poids.

22. Emulsion selon l'une quelconque des revendications précédentes, **caractérisée par le fait qu'**elle comprend au moins une charge.

23. Emulsion selon l'une quelconque des revendication précédentes, **caractérisée par le fait qu'**elle comprend au moins une matière colorante.

24. Emulsion selon l'une quelconque des revendications précédentes, **caractérisée par le fait qu'**elle comprend un ingrédient cosmétique choisi parmi les antioxydants, les parfums, les conservateurs, les neutralisants, les tensioactifs, les cires, les filtres solaires, les vitamines, les hydratants, les composés auto-bronzants, les actifs antirides.

25. Composition selon l'une quelconque des revendications précédentes, **caractérisée par le fait que** la composition est un fond de teint, un fard à paupières, un fard à joué, un produit anticernes, un produit de maquillage du corps, un rouge à lèvres, une base de soin pou la peau, une crème de soin, une composition de soin pour les lèvres; une composition de protection salaire, une composition autobronzante ; un déodorant.

26. Fond de teint comprenant une composition selon l'une quelconque des revendications 1 à 24

27. Procédé cosmétique de maquillage ou de soin non thérapeutique de la peau comprenant l'application sur la peau d'une composition selon l'une quelconque des revendications précédentes.

## Claims

1. Solid water-in-oil emulsion comprising an aqueous phase emulsified with an emulsifying surfactant C₈-C₂₂ alkyl dimethicone copolyol of formula (I) below: 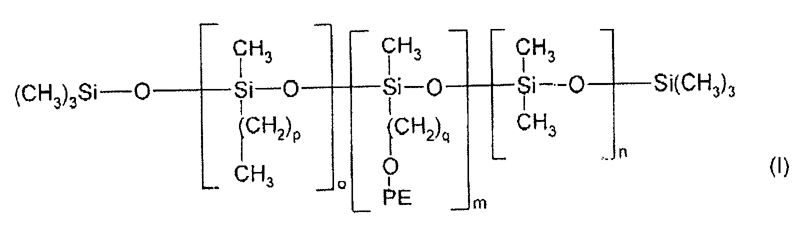 in which:
- PE represents (-C₂H₄O)ₓ-(C₃H₆O)_{y}-R, R being chosen from a hydrogen atom and an alkyl radical containing from 1 to 4 carbon atoms, x ranging from 0 to 100 and y ranging from 0 to 80, x and y not simultaneously being 0,
- m ranging from 1 to 40,
- n ranging from 10 to 200,
- o ranging from 1 to 100,
- p ranging from 7 to 21,
- q ranging from 0 to 4,
the said surfactant being present in a fatty phase comprising an oil and a wax, **characterized in that** the fatty phase comprises a block copolymer of ethylene oxide and/or of propylene oxide and of an alkylene oxide containing from 6 to 40 carbon atoms, the copolymer having a weight-average molecular weight ranging from 5000 to 8000, and **in that** the emulsion has a hardness such that the penetration force is greater than or equal to 40 g.

2. Emulsion according to Claim 1, **characterized in that** the alkylene oxide of the block copolymer contains from 6 to 30 carbon atoms, preferably from 8 to 20 carbon atoms, preferentially from 10 to 18 carbon atoms and more preferentially from 10 to 14 carbon atoms.

3. Emulsion according to Claim 1 or 2, **characterized in that** the alkylene oxide of the copolymer contains from 6 to 30 carbon atoms, preferably from 8 to 20 carbon atoms, preferentially from 10 to 18 carbon atoms and more preferentially from 10 to 14 carbon atoms.

4. Emulsion according to any one of the preceding claims, **characterized in that** the number-average molecular weight of the copolymer ranges from 5000 to 8000, preferably from 5500 to 7000, preferentially from 5500 to 6500 and more preferentially from 5800 to 6200.

5. Emulsion according to any one of the preceding claims, **characterized in that** the copolymer comprises from 35 to 55 ethylene oxide and/or propylene oxide units and from 15 to 30 alkylene oxide units containing from 6 to 40 carbon atoms.

6. Emulsion according to any one of the preceding claims, **characterized in that** the copolymer is such that the ratio between the number of ethylene oxide and/or propylene oxide units and the number of alkylene oxide units containing from 6 to 40 carbon atoms ranges from 1.5 to 2.5, preferably from 1.8 to 2.3 and preferentially from 1.9 to 2.1.

7. Emulsion according to any one of the preceding claims, **characterized in that** the copolymer is present in a content ranging from 1% to 25% by weight, preferably ranging from 1% to 18% by weight and preferentially ranging from 1% to 10% by weight relative to the total weight of the composition.

8. Emulsion according to any one of the preceding claims, **characterized in that** it has a hardness such that the penetration force ranges from 40 g to 150 g, preferably from 50 g to 120 g, preferentially from 50 g to 90 g and more preferentially from 60 g to 80 g.

9. Emulsion according to any one of the preceding claims, **characterized in that** the fatty phase comprises a volatile oil.

10. Emulsion according to Claim 9, **characterized in that** the volatile oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, isododecane, isodecane and isohexadecane, and mixtures thereof.

11. Emulsion according to Claim 9 or 10, **characterized in that** the volatile oil is present in a content ranging from 0.1% to 35% by weight, preferably ranging from 1% to 30% by weight and preferentially ranging from 5% to 25% by weight relative to the total weight of the composition.

12. Emulsion according to any one of the preceding claims, **characterized in that** the fatty phase comprises a non-volatile oil.

13. Emulsion according to Claim 12, **characterized in that** the non-volatile oil is present in a content ranging from 0.1% to 35% by weight, preferably ranging from 1% to 30% by weight and preferentially ranging from 5% to 25% by weight relative to the total weight of the composition.

14. Emulsion according to any one of the preceding claims, **characterized in that** the wax has a hardness ranging from 5 MPa to 9 MPa.

15. Emulsion according to any one of the preceding claims, **characterized in that** the wax has a hardness ranging from 6 MPa to 9 MPa and preferably ranging from 7 MPa to 9 MPa.

16. Emulsion according to any one of the preceding claims, **characterized in that** the wax is chosen from carnauba wax, microcrystalline waxes, ozokerites, hydrogenated jojoba oil, polyethylene waxes, poly(C₂₄-C₂₈)alkyl methyl dimethyl siloxane waxes, palm butter, C₂₀-C₄₀ alkyl stearate, stearyl benzoate and shellac wax, and mixtures thereof.

17. Emulsion according to any one of the preceding claims, **characterized in that** the wax is chosen from carnauba wax, ozokerites, hydrogenated jojoba oil and polyethylene waxes.

18. Emulsion according to any one of the preceding claims, **characterized in that** the wax is present in a content ranging from 1% to 10% by weight, preferably ranging from 2% to 7% by weight and preferentially ranging from 2.5% to 5% by weight relative to the total weight of the composition.

19. Emulsion according to any one of the preceding claims, **characterized in that** the aqueous phase is present in a content ranging from 30% to 50% by weight and preferably ranging from 35% to 45% by weight relative to the total weight of the emulsion.

20. Foundation according to any one of the preceding claims, **characterized in that** the aqueous phase comprises a water-miscible solvent chosen especially from C₂-C₄ primary alcohols, glycols and glycol ethers, and mixtures thereof.

21. Emulsion according to any one of the preceding claims, **characterized in that** the emulsifying surfactant is present in a content ranging from 1% to 10% by weight and preferably ranging from 2% to 5% by weight relative to the total weight of the composition.

22. Emulsion according to any one of the preceding claims, **characterized in that** it comprises at least one filler.

23. Emulsion according to any one of the preceding claims, **characterized in that** it comprises at least one dyestuff.

24. Emulsion according to any one of the preceding claims, **characterized in that** it comprises a cosmetic ingredient chosen from antioxidants, fragrances, preserving agents, neutralizers, surfactants, waxes, sunscreens, vitamins, moisturizers, self-tanning compounds and anti-wrinkle active agents.

25. Composition according to any one of the preceding claims, **characterized in that** the composition is a foundation, an eyeshadow, a makeup rouge, a concealer product, a body makeup product, a lipstick, a skincare base, a care cream; a lipcare composition; an antisun composition, a self-tanning composition; a deodorant.

26. Foundation comprising a composition according to any one of Claims 1 to 24.

27. Non-therapeutic cosmetic process for making up or caring for the skin, comprising the application to the skin of a composition according to any one of the preceding claims.

## Patentansprüche

1. Feste Wasser-in-Öl Emulsion, die eine wässrige Phase enthält, die mit einem grenzflächenaktiven, emulgierenden Alkyl(C₈-₂₂)-dimethiconcopolyol der folgenden Formel (I) emulgiert ist: 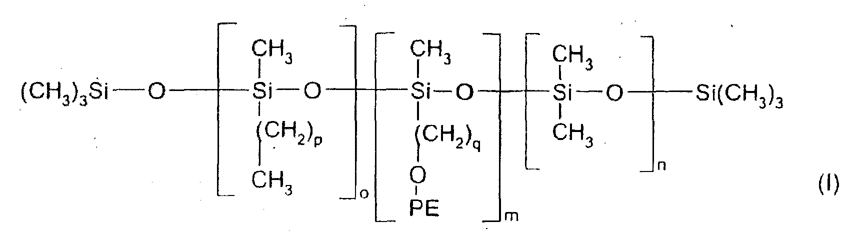 in der Formel:
• PE bedeutet (-C₂H₄O)ₓ-(-C₃H₆O)_{y}-R, wobei R unter einem Wasserstoffatom oder einer Alkylgruppe mit 1 bis 4 Kohlenstoffatomen ausgewählt ist, x im Bereich von 0 bis 100 und y im Bereich von 0 bis 80 liegt, wobei x und y nicht gleichzeitig Null bedeuten;
• m liegt im Bereich von 1 bis 40;
• n liegt im Bereich von 10 bis 200;
• o liegt im Bereich von 1 bis 100;
• p liegt im Bereich von 7 bis 21;
• q liegt im Bereich von 0 bis 4;
wobei der grenzflächenaktive Stoff in einer Fettphase vorliegt, die ein Öl und ein Wachs enthält, **dadurch gekennzeichnet, dass** die Fettphase ein Sequenzcopolymer von Ethylenoxid und/oder Propylenoxid und Alkylenoxid mit 6 bis 40 Kohlenstoffatomen enthält, wobei das Copolymer eine gewichtsmittlere Molmasse von 5000 bis 8000 aufweist, und **dadurch**, dass die Emulsion eine Härte besitzt, die so ist, dass die Penetrationskraft größer oder gleich 40 g ist.

2. Emulsion nach Anspruch 1, **dadurch gekennzeichnet, dass** das Alkylenoxid des Sequenzcopolymers 6 bis 30 Kohlenstoffatome, vorzugsweise 8 bis 20 Kohlenstoffatome, noch bevorzugter 10 bis 18 Kohlenstoffatome und besonders bevorzugt 10 bis 14 Kohlenstoffatome aufweist.

3. Emulsion nach Anspruch 1 oder 2, **dadurch gekennzeichnet, dass** das Alkylenoxid des Copolymers 6 bis 30 Kohlenstoffatome, vorzugsweise 8 bis 20 Kohlenstoffatome, noch bevorzugter 10 bis 18 Kohlenstoffatome und besonders bevorzugt 10 bis 14 Kohlenstoffatome aufweist.

4. Emulsion nach einem der vorhergehenden Ansprüche, **dadurch gekennzeichnet, dass** die zahlenmittlere Molmasse des Copolymers im Bereich von 5000 bis 8000, vorzugsweise 5500 bis 7000, bevorzugt 5500 bis 6500 und besonders bevorzugt 5800 bis 6200 liegt.

5. Emulsion nach einem der vorhergehenden Ansprüche, **dadurch gekennzeichnet, dass** das Copolymer 35 bis 55 Ethylenoxideinheiten und/oder Propylenoxideinheiten und 15 bis 30 Alkylenoxideinheiten mit 6 bis 40 Kohlenstoffatomen aufweist.

6. Emulsion nach einem der vorhergehenden Ansprüche, **dadurch gekennzeichnet, dass** das Copolymer so vorliegt, dass das Verhältnis der Anzahl der Ethylenoxideinheiten und/oder Propylenoxideinheiten und der Anzahl der Alkylenoxideinheiten mit 6 bis 40 Kohlenstoffatomen im Bereich von 1,5 bis 2,5, vorzugsweise 1,8 bis 2,3 und noch bevorzugter 1,9 bis 2,1 liegt.

7. Emulsion nach einem der vorhergehenden Ansprüche, **dadurch gekennzeichnet, dass** das Copolymer in einem Mengenanteil von 1 bis 25 Gew.-%, bezogen auf das Gesamtgewicht der Zusammensetzung, vorzugsweise 1 bis 18 Gew.-% und bevorzugt 1 bis 10 Gew.-% enthalten ist.

8. Emulsion nach einem der vorhergehenden Ansprüche, **dadurch gekennzeichnet, dass** sie eine Härte aufweist, die so ist, dass die Penetrationskraft im Bereich von 40 bis 150 g, vorzugsweise 50 bis 120 g, noch bevorzugter 50 bis 90 g und besonders bevorzugt 60 bis 80 g liegt.

9. Emulsion nach einem der vorhergehenden Ansprüche, **dadurch gekennzeichnet, dass** die Fettphase ein flüchtiges Öl enthält.

10. Emulsion nach Anspruch 9, **dadurch gekennzeichnet, dass** das flüchtige Öl unter Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan, Isododecan, Isodecan, Isohexadecan und deren Gemischen ausgewählt ist.

11. Emulsion nach Anspruch 9 oder 10, **dadurch gekennzeichnet, dass** das flüchtige Öl in einem Mengenanteil von 0,1 bis 35 Gew.-%, bezogen auf das Gesamtgewicht der Zusammensetzung, vorzugsweise 1 bis 30 Gew.-% und noch bevorzugter 5 bis 25 Gew.-% vorliegt.

12. Emulsion nach einem der vorhergehenden Ansprüche, **dadurch gekennzeichnet, dass** die Fettphase ein nicht flüchtiges Öl enthält.

13. Emulsion nach Anspruch 12, **dadurch gekennzeichnet, dass** das nicht flüchtige Öl in einem Mengenanteil von 0,1 bis 35 Gew.-%, bezogen auf das Gesamtgewicht der Zusammensetzung, vorzugsweise 1 bis 30 Gew.-% und noch bevorzugter 5 bis 25 Gew.-% vorliegt.

14. Emulsion nach einem der vorhergehenden Ansprüche, **dadurch gekennzeichnet, dass** das Wachs eine Härte von 5 bis 9 MPa aufweist.

15. Emulsion nach einem der vorhergehenden Ansprüche, **dadurch gekennzeichnet, dass** das Wachs eine Härte von 6 bis 9 MPa und vorzugsweise 7 bis 9 MPa aufweist.

16. Emulsion nach einem der vorhergehenden Ansprüche, **dadurch gekennzeichnet, dass** das Wachs unter Carnaubawachs, mikrokristallinen Wachsen, Ozokeriten, hydriertem Jojobaöl, Polyethylenwachsen, Polyalkyl(C₂₄-₂₈)methyldimethylsiloxanwachsen, Palmbutter, Alkyl(C₂₀-₄₀)stearat, Stearylbenzoat, Schellack und deren Gemischen ausgewählt ist.

17. Emulsion nach einem der vorhergehenden Ansprüche, **dadurch gekennzeichnet, dass** das Wachs unter Carnaubawachs, Ozokeriten, hydriertem Jojobaöl und Polyethylenwachsen ausgewählt ist.

18. Emulsion nach einem der vorhergehenden Ansprüche, **dadurch gekennzeichnet, dass** das Wachs in einem Mengenanteil von 1 bis 10 Gew.-%, bezogen auf das Gesamtgewicht der Zusammensetzung, vorzugsweise 2 bis 7 Gew.-% und noch bevorzugter 2,5 bis 5 Gew.-% vorliegt.

19. Emulsion nach einem der vorhergehenden Ansprüche, **dadurch gekennzeichnet, dass** die wässrige Phase in einem Mengenanteil von 30 bis 50 Gew.-% und vorzugsweise 35 bis 45 Gew.-%, bezogen auf das Gesamtgewicht der Emulsion, enthalten ist.

20. Make-up nach einem der vorhergehenden Ansprüche, **dadurch gekennzeichnet, dass** die wässrige Phase ein mit Wasser mischbares Lösemittel enthält, das insbesondere unter den primären Alkoholen mit 2 bis 4 Kohlenstoffatomen, Glycolen, Glycolethern und deren Gemischen ausgewählt ist.

21. Emulsion nach einem der vorhergehenden Ansprüche, **dadurch gekennzeichnet, dass** der emulgierende grenzflächenaktive Stoff in einem Mengenanteil von 1 bis 10 Gew.-%, bezogen auf das Gesamtgewicht der Zusammensetzung, und vorzugsweise 2 bis 5 Gew.-% enthalten ist.

22. Emulsion nach einem der vorhergehenden Ansprüche, **dadurch gekennzeichnet, dass** sie mindestens einen Füllstoff enthält.

23. Emulsion nach einem der vorhergehenden Ansprüche, **dadurch gekennzeichnet, dass** sie mindestens ein Farbmittel enthält.

24. Emulsion nach einem der vorhergehenden Ansprüche, **dadurch gekennzeichnet, dass** sie einen kosmetischen Bestandteil enthält, der unter den Antioxidantien, Parfums, Konservierungsmitteln, Neutralisationsmitteln, grenzflächenaktiven Stoffen, Wachsen, Sonnenschutzfiltern, Vitaminen, Hydratisierungsmitteln, Selbstbräunungsmitteln und Wirkstoffen gegen Falten ausgewählt ist.

25. Zusammensetzung nach einem der vorhergehenden Ansprüche, **dadurch gekennzeichnet, dass** es sich bei der Zusammensetzung um ein Make-up, einen Lidschatten, ein Wangenrouge, ein Produkt gegen Augenringe, ein Produkt zum Schminken des Körpers, einen Lippenstift, eine Pflegebasis für die Haut, eine Pflegecreme; eine Zusammensetzung für die Pflege der Lippen, eine Zusammensetzung für den Sonnenschutz, ein Selbstbräunungsmittel; ein Deodorant handelt.

26. Make-up, das eine Zusammensetzung nach einem der Ansprüche 1 bis 24 umfasst.

27. Kosmetisches Verfahren zum Schminken oder für die nicht therapeutische Pflege der Haut, das das Aufbringen einer Zusammensetzung nach einem der vorhergehenden Ansprüche auf die Haut umfasst.
